# EUROPEAN PATENT SPECIFICATION

(11) **EP 4 062 882 B1**
(45) Date of publication and mention of the grant of the patent: **31.12.2025**
(21) Application number: 21164142.8
(22) Date of filing: 23.03.2021
(51) Int. Cl.: A61F 9/02

(54) **GOGGLE AND ANGLE ADJUSTMENT MECHANISM THEREOF**
BRILLE UND WINKELVERSTELLMECHANISMUS DAFÜR
LUNETTES ET MÉCANISME DE RÉGLAGE D'ANGLE CORRESPONDANT

(43) Date of publication of application: 28.09.2022
(73) Proprietor: Aswan International Corp., Taipei City 100 (TW)
(72) Inventor: CHIN, Meng- Hsien, 115 TAIPEI CITY (TW)
(74) Representative: Wunderlich & Heim Patentanwälte PartG mbB

(56) References cited:
- DE-U1- 20 207 349
- US-A- 5 812 234

## Description

### FIELD OF THE DISCLOSURE

The present disclosure relates to a goggle, and more particularly to a goggle and an angle adjustment mechanism thereof for adjusting an angle of at least one temple thereof.

### BACKGROUND OF THE DISCLOSURE

A conventional goggle needs to have an adjustment function for matching different facial sizes and contours of users. For example, the conventional goggle includes a frame and two temple structures that are assembled to the frame, and each of the two temple structures is formed by assembling a plurality of components, thereby providing an angle adjustment function. However, the temple structure of the conventional goggle is manufactured by a complex assembling process and is easily damaged, which results in a high manufacturing cost of the conventional goggle.

US 5 812 234 A discloses an improved eyeglass construction, which is described having a novel means for varying the inclination of the lens relative to the temples and for adjusting the length of the temples, which involves an enclosed pair of mating ribbed arcuate segments for adjusting the temples relative to the lens.

### SUMMARY OF THE DISCLOSURE

In response to the above-referenced technical inadequacy, the present disclosure provides an angle adjustment mechanism of a goggle with the features of claim 1 and a goggle with the features of claim 7 provided with the angle adjustment mechanism to effectively improve on the issues associated with conventional goggles.

In one aspect, the present invention provides the goggle, which includes a lens, two lateral frames, a top frame, and two bendable temples. The two lateral frames are respectively connected to two opposite sides of the lens. Two sides of the two lateral frames away from each other each have an assembling slot. The assembling slot of each of the two lateral frames has a retaining rack arranged therein, and the retaining rack is in an arced shape and is arranged adjacent to the lens. The top frame is connected to the lens and the two lateral frames. The lens, the two lateral frames, and the top frame jointly define an eye-protection space. Each of the two bendable temples is integrally formed as a single one-piece structure and includes an assembling segment having an engaging tooth and a flexible segment that extends from the assembling segment. The assembling segments of the two bendable temples are respectively and pivotally connected to the assembling slots of the two lateral frames, and each of the two bendable temples and the corresponding assembling slot are jointly defined as an angle adjustment mechanism. In each of the two angle adjustment mechanisms, the engaging tooth of the assembling segment is inserted into the assembling slot and is engaged with the retaining rack, and the assembling segment is rotatable relative to the assembling slot in an angle range, so that the engaging tooth is engaged with and movable from one end of the retaining rack to another end of the retaining rack.

In another aspect, the present invention provides the angle adjustment mechanism of a goggle, which inter alia includes an assembling slot and a bendable temple. The assembling slot has a retaining rack arranged therein. The retaining rack is in an arced shape. The bendable temple is integrally formed as a single one-piece structure and includes an assembling segment pivotally connected to the assembling slot, and a flexible segment that extends from the assembling segment, wherein the assembling segment has an engaging tooth.

The engaging tooth of the assembling segment is inserted into the assembling slot and is engaged with the retaining rack, and the assembling segment is rotatable relative to the assembling slot in an angle range, so that the engaging tooth is engaged with and movable from one end of the retaining rack to another end of the retaining rack.

Therefore, the goggle of the present disclosure is provided with the angle adjustment mechanism that is structurally simplified (e.g., the bendable temple formed as a single one-piece structure is pivotally connected to the assembling slot through the assembling segment, and the engaging tooth is movably engaged with the retaining rack), thereby effectively reducing manufacturing cost thereof and probability of being damaged.

These and other aspects of the present disclosure will become apparent from the following description of the embodiment taken in conjunction with the following drawings and their captions, although variations and modifications therein may be affected without departing from the scope of the novel concepts of the disclosure.

### BRIEF DESCRIPTION OF THE DRAWINGS

The described embodiments may be better understood by reference to the following description and the accompanying drawings, in which:
FIG. 1 is a perspective view of a goggle according to a first embodiment of the present disclosure;
FIG. 2 is a perspective view showing the goggle of FIG. 1 from another angle of view;
FIG. 3 is a partial exploded view of FIG. 1;
FIG. 4 is a perspective cross-sectional view of FIG. 3;
FIG. 5 is a planar view of FIG. 3;
FIG. 6 is a planar view of FIG. 4;
FIG. 7 is a perspective cross-sectional view showing a portion of the goggle according to the first embodiment of the present disclosure;
FIG. 8 is a planar view of FIG. 7;
FIG. 9 is a planar cross-sectional view showing a portion of the goggle according to the first embodiment of the present disclosure;
FIG. 10 is a planar view showing a bendable temple upwardly rotated relative to a lens assembly according to the first embodiment of the present disclosure;
FIG. 11 is a planar view showing the bendable temple downwardly rotated relative to the lens assembly according to the first embodiment of the present disclosure;
FIG. 12 is an exploded view of a goggle according to a second embodiment of the present disclosure; and
FIG. 13 is a planar view of the goggle according to the second embodiment of the present disclosure.

### DETAILED DESCRIPTION OF THE EXEMPLARY EMBODIMENTS

The present disclosure is more particularly described in the following examples that are intended as illustrative only since numerous modifications and variations therein will be apparent to those skilled in the art. Like numbers in the drawings indicate like components throughout the views. As used in the description herein and throughout the claims that follow, unless the context clearly dictates otherwise, the meaning of "a", "an", and "the" includes plural reference, and the meaning of "in" includes "in" and "on". Titles or subtitles can be used herein for the convenience of a reader, which shall have no influence on the scope of the present disclosure.

The terms used herein generally have their ordinary meanings in the art. In the case of conflict, the present document, including any definitions given herein, will prevail. The same thing can be expressed in more than one way. Alternative language and synonyms can be used for any term(s) discussed herein, and no special significance is to be placed upon whether a term is elaborated or discussed herein. A recital of one or more synonyms does not exclude the use of other synonyms. The use of examples anywhere in this specification including examples of any terms is illustrative only, and in no way limits the scope and meaning of the present disclosure or of any exemplified term. Likewise, the present disclosure is not limited to various embodiments given herein. Numbering terms such as "first", "second" or "third" can be used to describe various components, signals or the like, which are for distinguishing one component/signal from another one only, and are not intended to, nor should be construed to impose any substantive limitations on the components, signals or the like.

### [First Embodiment]

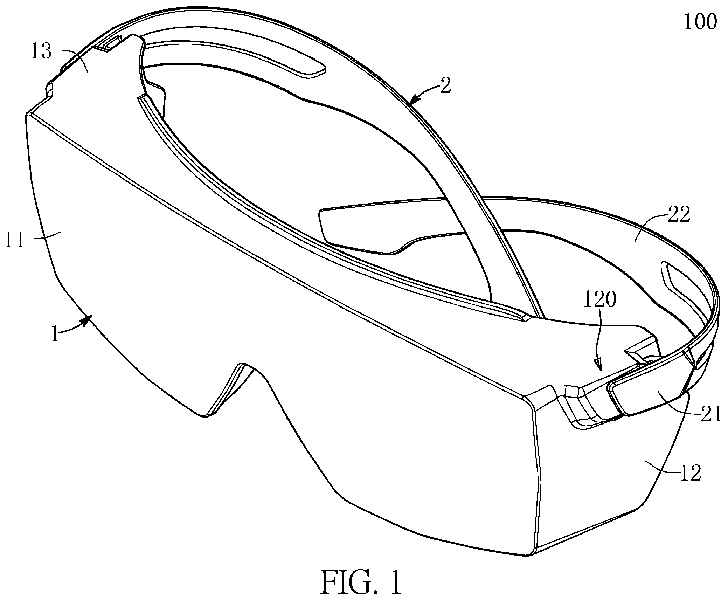

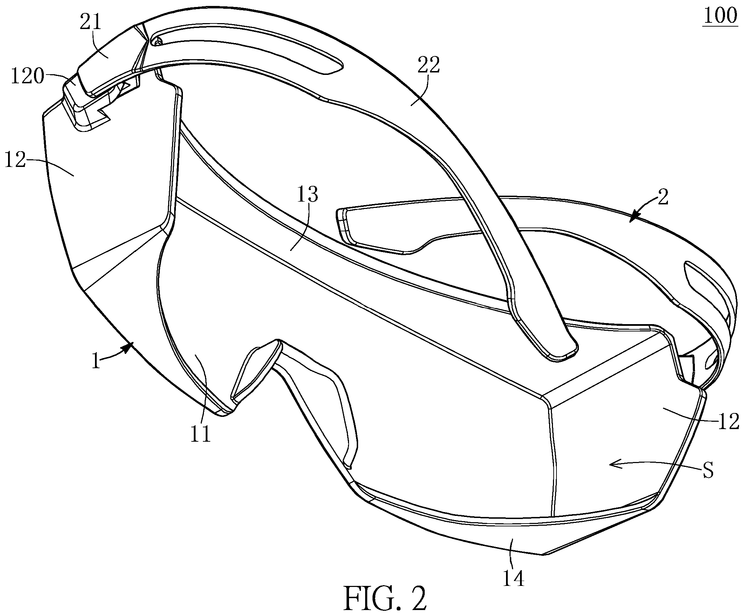

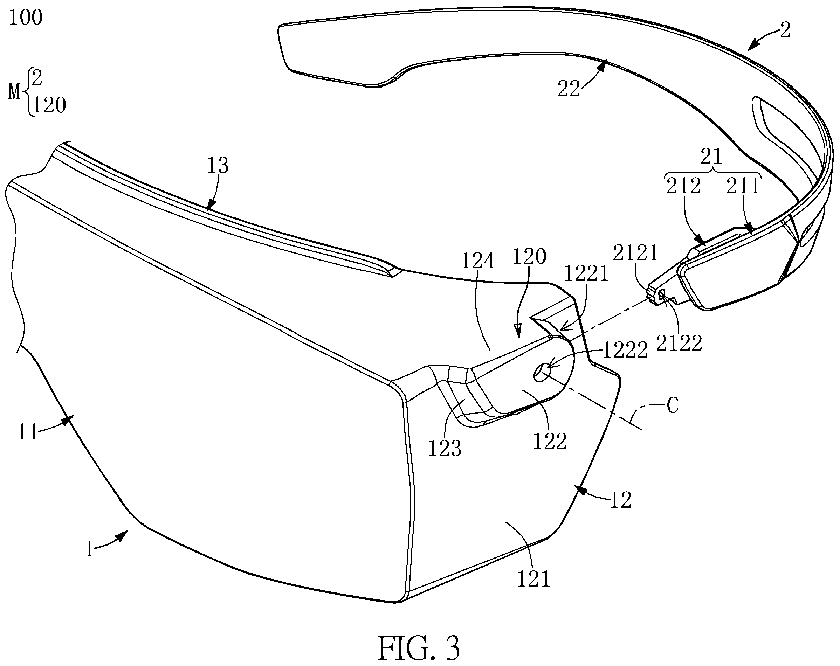

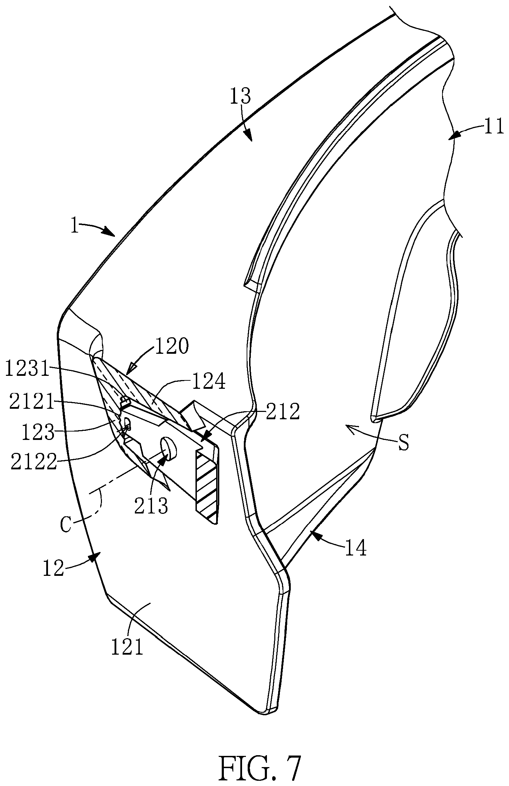

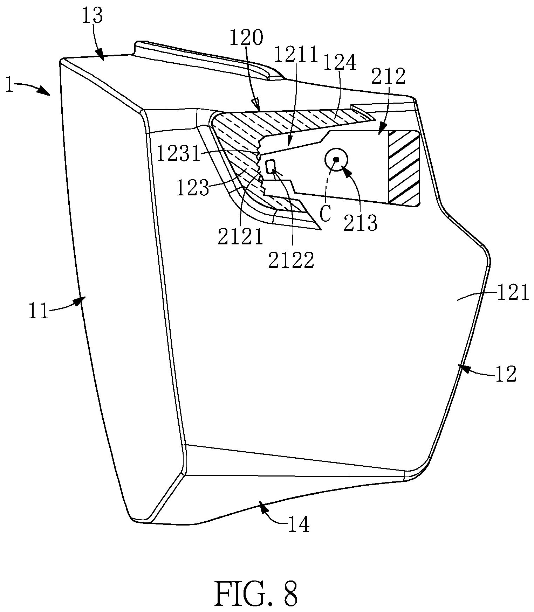

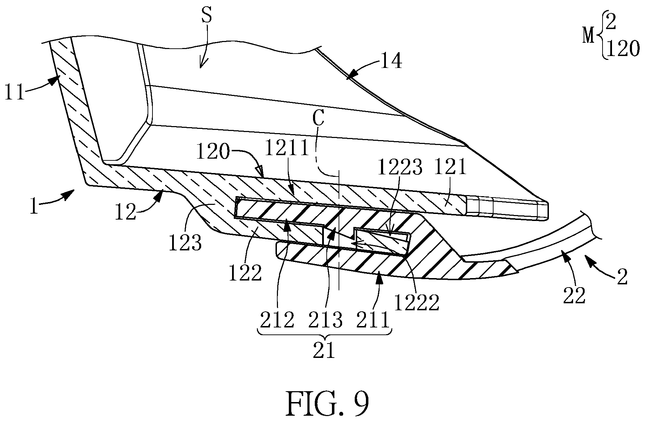

Referring to FIG. 1 to FIG. 11, a first embodiment of the present disclosure provides a goggle 100. As shown in FIG. 1 and FIG. 2, the goggle 100 in the present embodiment is used for being worn on (e.g., attached to) a user's face to protect the user's face (e.g., the user's eyes). The goggle 100 in the present embodiment includes a lens assembly 1 and two bendable temples 2 that are detachably assembled to the lens assembly 1. The following description describes the structure and connection relationship of each of the lens assembly 1 and the two bendable temples 2.

As shown in FIG. 1 to FIG. 3, the lens assembly 1 includes a lens 11, two lateral frames 12 respectively connected to two opposite sides of the lens 11, a top frame 13 connected to a top edge of the lens 11 and the two lateral frames 12, and a bottom frame 14 that is connected to a bottom edge of the lens 11 and the two lateral frames 12. The lens 11, the two lateral frames 12, the top frame 13, and the bottom frame 14 jointly define an eye-protection space S.

It should be noted that the lens assembly 1 (e.g., the lens 11, the two lateral frames 12, the top frame 13, and the bottom frame 14) in the present embodiment is light-permeable and is integrally formed as a single one-piece structure, but the present disclosure is not limited thereto. For example, in other embodiments of the present disclosure not shown in the drawings, the lens assembly 1 can be provided without the bottom frame 14; or, the lens assembly 1 can be provided by assembling a plurality of components (e.g., the lens 11 can be assembled in an annular frame that is formed by the two lateral frames 12, the top frame 13, and the bottom frame 14).

Two sides of the two lateral frames 12 away from each other each have an assembling slot 120. As the two lateral frames 12 in the present embodiment are of the substantially same structure and are mirror-symmetrical to each other, the following description discloses the structure of just one of the two lateral frames 12 for the sake of brevity, but the present disclosure is not limited thereto. For example, in other embodiments of the present disclosure not shown in the drawings, the two lateral frames 12 can be of different structures.

The lateral frame 12 in the present embodiment includes a main board 121, a limiting lateral board 122 facing the main board 121, and a limiting end portion 123 connecting the main board 121 and the limiting lateral board 122, and a limiting top board 124 that is coplanar with the top frame 13. The limiting top board 124 in the present embodiment is integrally connected to the main board 121, the limiting end portion 123, and the limiting lateral board 122.

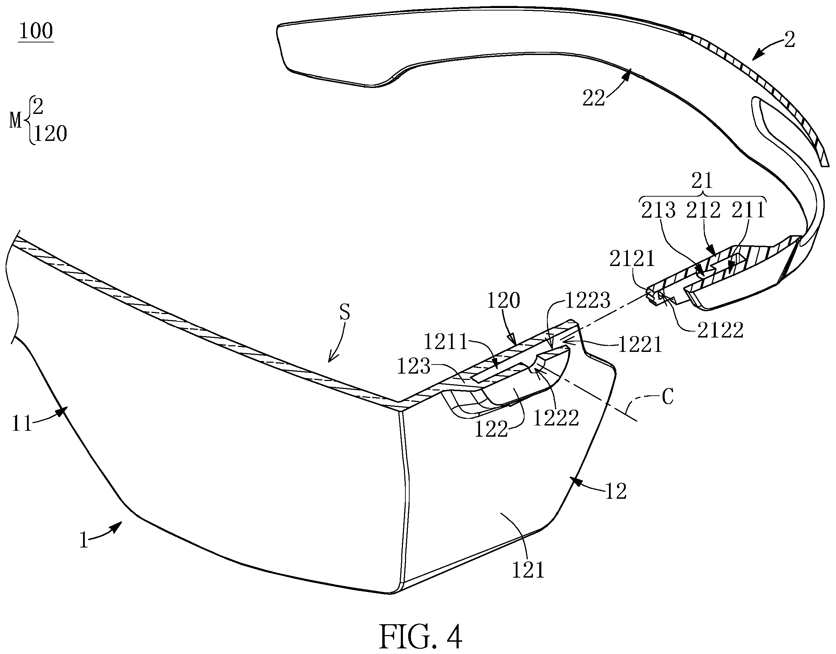

As shown in FIG. 4 to FIG. 6, the assembling slot 120 includes the limiting lateral board 122, the limiting end portion 123, the limiting top board 124, and a mating portion 1211 of the main board 121 that faces the limiting lateral board 122. In other words, a bottom of the assembling slot 120 in the present embodiment is a hollow region, thereby preventing any attachment (e.g., a chemical agent) from existing in the assembling slot 120 after the assembling slot 120 is formed, but the present disclosure is not limited thereto. For example, in other embodiments of the present disclosure not shown in the drawings, the lateral frame 12 (or the assembling slot 120) can be provided without the limiting top board 124, so that a top and a bottom of the assembling slot 120 are hollow regions.

Specifically, the assembling slot 120 has a retaining rack 1231 arranged therein, and the retaining rack 1231 is in an arced shape and is arranged adjacent to the lens 11. The assembling slot 120 has a slot opening 1221 arranged on an end of the limiting lateral board 122 away from the limiting end portion 123. The retaining rack 1231 is formed on the limiting end portion 123 and faces the slot opening 1221.

Moreover, the limiting lateral board 122 has a shaft hole 1222. The limiting lateral board 122 has a guiding groove 1223 recessed from the end thereof toward the shaft hole 1222. The guiding groove 1223 is in spatial communication with the slot opening 1221, and a distance between the guiding groove 1223 and the mating portion 1211 gradually reduces in a direction from the slot opening 1221 to the shaft hole 1222. The shaft hole 1222 in the present embodiment is formed by penetrating the limiting lateral board 122 along an axis C, and the retaining rack 1231 has a center of circle located at the axis C, but the present disclosure is not limited thereto. For example, in other embodiments of the present disclosure not shown in the drawings, the shaft hole 1222 can be recessed in an inner surface of the limiting lateral board 122, but the shaft hole 1222 does not penetrate through the limiting lateral board 122.

The assembling slots 120 of the lens assembly 1 in the present embodiment are respectively assembled to the two bendable temples 2, but the present disclosure is not limited thereto. Each of the two bendable temples 2 and the corresponding assembling slot 120 are jointly defined as an angle adjustment mechanism M. In other embodiments of the present disclosure not shown in the drawings, the angle adjustment mechanism M can be applied to a structure other than the lens assembly 1 shown in the drawings of the present embodiment according to design requirements.

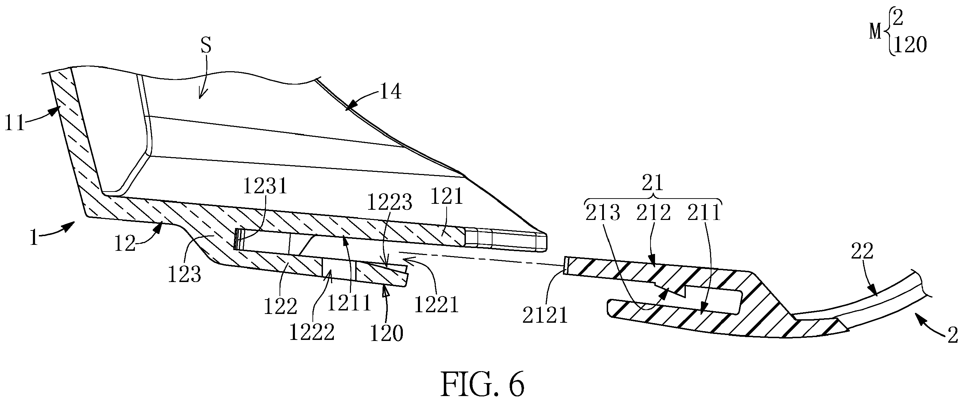

Specifically, as shown in FIG. 6 to FIG. 8, each of the two bendable temples 2 is integrally formed as a single one-piece structure, and each of the two bendable temples 2 in the present embodiment is made of a single material, but the present disclosure is not limited thereto. Each of the two bendable temples 2 includes an assembling segment 21 and a flexible segment 22 that extends from the assembling segment 21. The two assembling segments 21 are respectively and pivotally connected to the assembling slots 120 of the two lateral frames 12. The flexible segment 22 of each of the two bendable temples 2 curvedly extends from the assembling segment 21 toward the lens 11 (as shown in FIG. 1), so that the flexible segments 22 of the two bendable temples 2 of the goggle 100 can contact with each other.

As the two bendable temples 2 in the present embodiment are of the substantially same structure and are mirror-symmetrical to each other with respect to the lens assembly 1, the following description discloses the structure of just one of the two bendable temples 2 (and the corresponding angle adjustment mechanism M) for the sake of brevity, but the present disclosure is not limited thereto. For example, in other embodiments of the present disclosure not shown in the drawings, the two bendable temples 2 can be of different structures.

As shown in FIG. 6 to FIG. 9, the assembling segment 21 in the present embodiment includes a shielding sheet 211 connected to the flexible segment 22, an L-shaped arm 212 connected to an inner side of the shielding sheet 211, and a rotation shaft 213 that is formed on an inner side of the L-shaped arm 212. In other words, the rotation shaft 213 is located between the L-shaped arm 212 and the shielding sheet 211. Moreover, a part of the L-shaped arm 212 is inserted into the assembling slot 120, the rotation shaft 213 of the assembling segment 21 is assembled to the shaft hole 1222, the L-shaped arm 212 and the shielding sheet 211 jointly sandwich the limiting lateral board 122, and the shielding sheet 211 covers the shaft hole 1222.

Specifically, while the L-shaped arm 212 is inserted into the assembling slot 120, the rotation shaft 213 is moved along the guiding groove 1223 to be assembled to the shaft hole 1222, so that the assembling segment 21 can be pivotally connected to the assembling slot 120 along the axis C, but the present disclosure is not limited thereto.

Moreover, the L-shaped arm 212 of the assembling segment 21 has an engaging tooth 2121 arranged on a free end thereof, and the L-shaped arm 212 preferably has a thru-hole 2122 arranged adjacent to the engaging tooth 2121. A distance between the thru-hole 2122 and the rotation shaft 213 is at least three times of a distance between the thru-hole 2122 and the engaging tooth 2121. Accordingly, the thru-hole 2122 of the L-shaped arm 212 can provide a buffering function to the engaging tooth 2121.

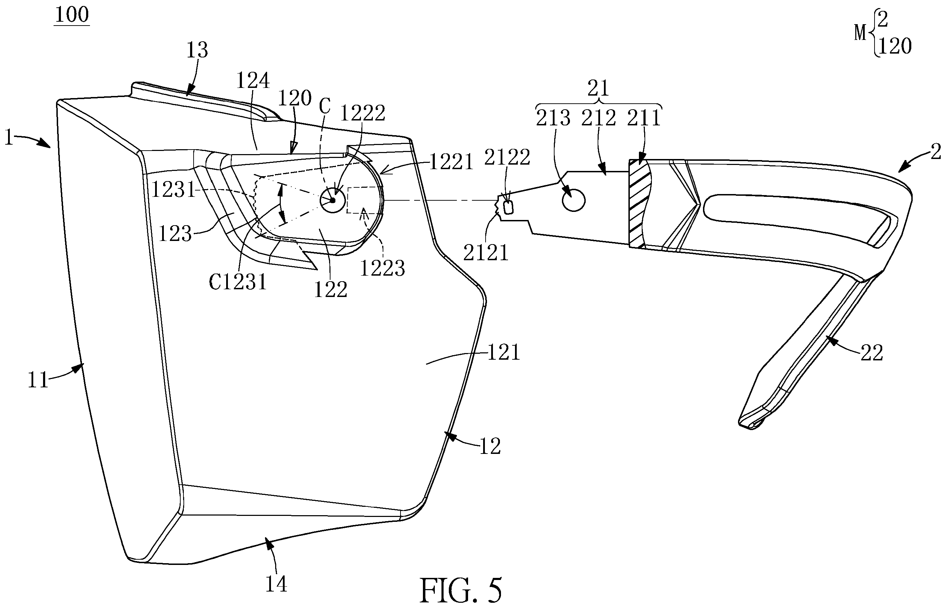

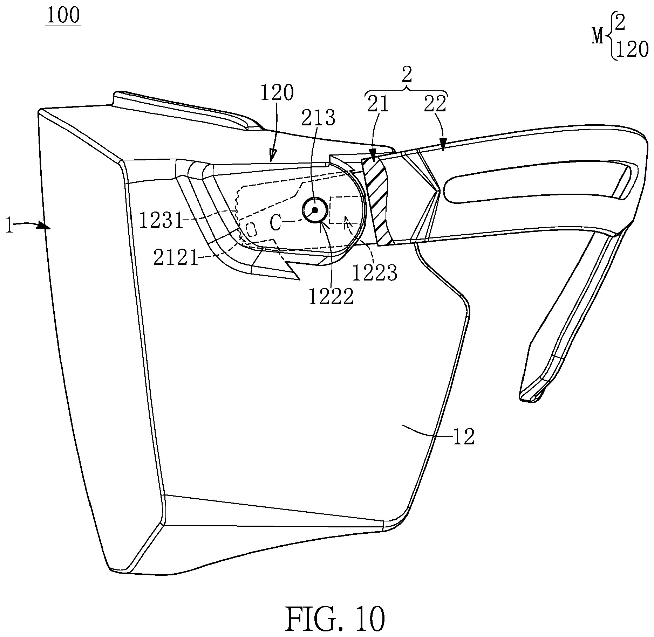

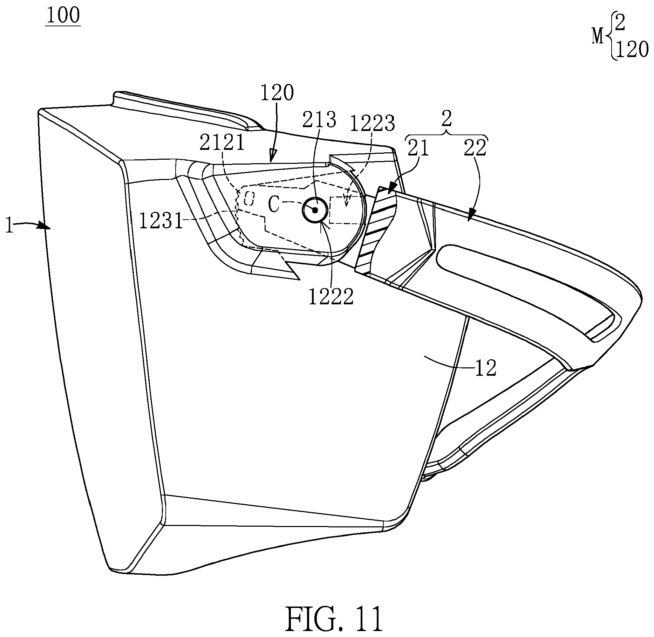

Specifically, as shown in FIG. 10 and FIG. 11, the engaging tooth 2121 of the assembling segment 21 is inserted into the assembling slot 120 and is engaged with the retaining rack 1231, and the assembling segment 21 is rotatable relative to the assembling slot 120 in an angle range, so that the engaging tooth 2121 is engaged with and movable from one end of the retaining rack 1231 to another end of the retaining rack 1231. In the present embodiment, as shown in FIG. 5, the retaining rack 1231 has a central angle C1231 with respect to the axis C, and the central angle is substantially equal to the angle range that is within a range from 15 degrees to 30 degrees.

Accordingly, the goggle 100 of the present embodiment is provided with the angle adjustment mechanism M that is structurally simplified (e.g., the bendable temple 2 formed as a single one-piece structure is pivotally connected to the assembling slot 120 through the assembling segment 21, and the engaging tooth 2121 is movably engaged with the retaining rack 1231), thereby effectively reducing manufacturing cost thereof and probability of being damaged.

In addition, the assembling segment 21 in the present embodiment is formed as a structure shown in the drawings, but the present disclosure is not limited thereto. For example, in other embodiments of the present disclosure not shown in the drawings, a portion of the assembling segment 21 other than the engaging tooth 2121 can be omitted according to design requirements (e.g., the shielding sheet 211 or the thru-hole 2122 can be omitted); or, the rotation shaft 213 of the assembling segment 21 can be replaced by a slot or a hole, and the shaft hole 1222 of the assembling slot 120 can be replaced by a protrusion the corresponds in shape to the slot or the hole.

### [Second Embodiment]

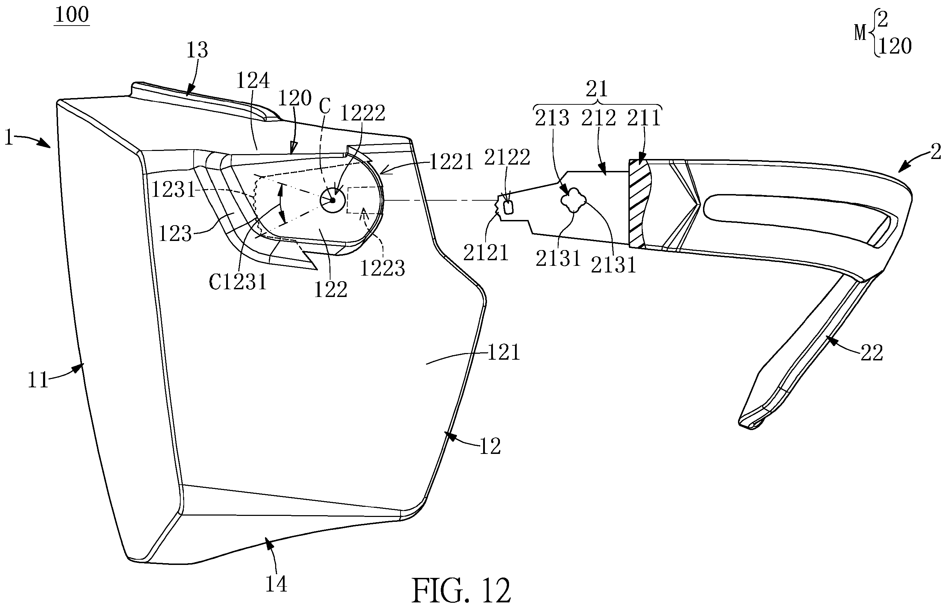

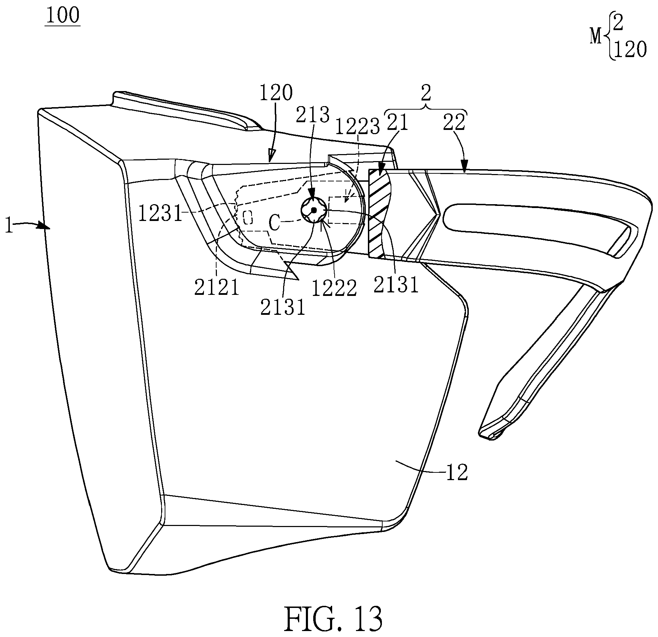

Referring to FIG. 12 and FIG. 13, a second embodiment of the present disclosure is similar to the first embodiment of the present disclosure. For the sake of brevity, descriptions of the same components in the first and second embodiments of the present disclosure will be omitted herein, and the following description only discloses different features between the first and second embodiments.

In the present embodiment, the rotation shaft 213 has a plurality of protrusions 2131 abutting against a wall of the shaft hole 1222. When the assembling segment 21 is rotated relative to the assembling slot 120, the protrusions 2131 are configured to scrape away any attachment (e.g., the chemical agent described in the first embodiment) on the wall of the shaft hole 1222, thereby keeping the rotation shaft 213 to smoothly rotate in the shaft hole 1222.

### [Beneficial Effects of the Embodiments]

In conclusion, the goggle of the present disclosure is provided with the angle adjustment mechanism that is structurally simplified (e.g., the bendable temple formed as a single one-piece structure is pivotally connected to the assembling slot through the assembling segment, and the engaging tooth is movably engaged with the retaining rack), thereby effectively reducing manufacturing cost thereof and probability of being damaged.

The foregoing description of the exemplary embodiments of the disclosure has been presented only for the purposes of illustration and description and is not intended to be exhaustive or to limit the disclosure to the precise forms disclosed. Many modifications and variations are possible in light of the above teaching.

The embodiments were chosen and described in order to explain the principles of the disclosure and their practical application so as to enable others skilled in the art to utilize the disclosure and various embodiments and with various modifications as are suited to the particular use contemplated. Alternative embodiments will become apparent to those skilled in the art to which the present disclosure pertains without departing from its scope.

## Claims

1. An angle adjustment mechanism (M) of a goggle, comprising:
an assembling slot (120) of a lateral frame (12) of the goggle, the assembling slot (120) having a retaining rack (1231) arranged therein, wherein the retaining rack (1231) is in an arced shape; and
a bendable temple (2) being integrally formed as a single one-piece structure and including an assembling segment (21) pivotally connected to the assembling slot (120) along an axis (C) that is horizontal in a normal wearing position of the goggle and having an engaging tooth (2121), and a flexible segment (22) that extends from the assembling segment (21);
wherein the engaging tooth (2121) of the assembling segment (21) is inserted into the assembling slot (120) and is engaged with the retaining rack (1231), and the assembling segment (21) is rotatable relative to the assembling slot (120) in an angle range, so that the engaging tooth (2121) is engaged with and movable from one end of the retaining rack (1231) to another end of the retaining rack (123 1);
wherein the assembling slot (120) includes a limiting lateral board (122), a mating portion (1211) facing the limiting lateral board (122), a limiting end portion (123) that connects the mating portion (1211) and the limiting lateral board (122), wherein the retaining rack (1231) is formed on the limiting end portion (123), the limiting lateral board (122) has a shaft hole (1222), and the assembling segment (21) has a rotation shaft (213) assembled to the shaft hole (1222); and
wherein the rotation shaft (213) has a plurality of protrusions (2131) abutting against a wall of the shaft hole (1222), and when the assembling segment (21) is rotated relative to the assembling slot (120), the protrusions (2131) are configured to scrape away any attachment on the wall of the shaft hole (1222).

2. The angle adjustment mechanism (M) according to claim 1, wherein the retaining rack (1231) has a center of circle located at the axis (C), the retaining rack (1231) has a central angle (C1231) with respect to the axis (C), and the central angle (C1231) is substantially equal to the angle range, and wherein the bendable temple (2) is made of a single material, and the angle range is within a range from 15 degrees to 30 degrees.

3. The angle adjustment mechanism (M) according to claim 1, wherein the assembling slot (120) has a slot opening (1221) arranged on an end of the limiting lateral board (122) away from the limiting end portion (123), wherein the limiting lateral board (122) has a guiding groove (1223) recessed from the end thereof toward the shaft hole (1222), the guiding groove (1223) is in spatial communication with the slot opening (1221), and the rotation shaft (213) is assembled into the shaft hole (1222) by being moved along the guiding groove (1223), and wherein a distance between the guiding groove (1223) and the mating portion (1211) gradually reduces in a direction from the slot opening (1221) to the shaft hole (1222).

4. The angle adjustment mechanism (M) according to claim 3, wherein the assembling segment (21) includes an L-shaped arm (212) inserted into the assembling slot (120), the engaging tooth (2121) is arranged on a free end of the L-shaped arm (212), and the rotation shaft (213) is formed on an inner side of the L-shaped arm (212).

5. The angle adjustment mechanism (M) according to claim 4, wherein the assembling segment (21) includes a shielding sheet (211), the L-shaped arm (212) is connected to the shielding sheet (211) so as to jointly sandwich the limiting lateral board (122), and the shielding sheet (211) covers the shaft hole (1222).

6. The angle adjustment mechanism (M) according to claim 5, wherein the L-shaped arm (212) has a thru-hole (2122) arranged adjacent to the engaging tooth (2121), and a distance between the thru-hole (2122) and the rotation shaft (213) is at least three times of a distance between the thru-hole (2122) and the engaging tooth (2121).

7. A goggle, comprising:
a lens (11);
two angle adjustment mechanisms (M) according to any one of claims 1 to 6, wherein two lateral frames (12) are respectively connected to two opposite sides of the lens (11), wherein two sides of the two lateral frames (12) away from each other each have the assembling slot (120) of the corresponding angle adjustment mechanisms (M), and the retaining rack (1231) is arranged adjacent to the lens (11); and
a top frame (13) connected to the lens (11) and the two lateral frames (12), wherein the lens (11), the two lateral frames (12), and the top frame (13) jointly define an eye-protection space (S),
wherein each of the two lateral frames (12) includes a main board (121), the limiting lateral board (122) of the corresponding angle adjustment mechanisms (M) facing the main board (121), and wherein the limiting end portion (123) of the corresponding angle adjustment mechanisms (M) connects the main board (121) and the limiting lateral board (122).

8. The goggle according to claim 7, wherein each of the two lateral frames (12) includes a limiting top board (124) coplanar with the top frame (13), and wherein in each of the two lateral frames (12), the limiting top board (124) is integrally connected to the main board (121), the limiting end portion (123), and the limiting lateral board (122).

9. The goggle according to claim 7, wherein each of the two bendable temples (2) is made of a single material, and wherein the flexible segment (22) of each of the two bendable temples (2) curvedly extends from the assembling segment (21) toward the lens (11), so that the flexible segments (22) of the two bendable temples (2) contact each other, and wherein the lens, the two lateral frames (12), and the top frame (13) are light-permeable and are integrally formed as a single one-piece structure, and the angle range is within a range from 15 degrees to 30 degrees.

## Patentansprüche

1. Winkeleinstellmechanismus (M) einer Schutzbrille, der aufweist:
ein Montageschlitz (120) eines Seitenrahmens (12) der Schutzbrille, wobei der Montageschlitz (120) ein darin angeordnetes Haltegestell (1231) aufweist, wobei das Haltegestell (1231) in einer gebogenen Form ist; und
einen biegsamen Bügel (2), der integral ausgebildet ist als eine einteilige Struktur und ein Montagesegment (21) aufweist, das schwenkbar mit dem Montageschlitz (120) entlang einer Achse (C) verbunden ist, die in normaler Trageposition der Schutzbrille horizontal verläuft, und einen Eingriffszahn (2121), sowie ein flexibles Segment (22) aufweist, das sich von dem Montagesegment (21) erstreckt;
wobei der Eingriffszahn (2121) des Montagesegments (21) in den Montageschlitz (120) eingeführt ist und mit dem Haltegestell (1231) in Eingriff steht, und das Montagesegment (21) relativ zum Montageschlitz (120) in einem Winkelbereich drehbar ist, so dass der Eingriffszahn (2121) mit dem Haltegestell (1231) in Eingriff steht und von einem Ende des Haltegestells (1231) zum anderen Ende des Haltegestells (1231) beweglich ist;
wobei der Montageschlitz (120) eine begrenzende Seitenplatte (122), einem, der begrenzenden Seitenplatte (122) zugewandten, passenden Abschnitt (1211), einen begrenzenden Endabschnitt (123) aufweist, der den passenden Abschnitt (1211) und die begrenzende Seitenplatte (122) verbindet, wobei das Haltegestell (1231) am begrenzenden Endabschnitt (123) gebildet ist, die begrenzende Seitenplatte (122) eine Schaftloch (1222) aufweist, und das Montagesegment (21) einen Rotationswelle (213) besitzt, der in dem Schaftloch (1222) montiert ist; und
wobei der Rotationswelle (213) weist mehrere Vorsprünge (2131) hat, die gegen eine Wand des Schaftloches (1222) anliegen, und wenn das Montagesegment (21) relativ zum Montageschlitz (120) gedreht wird, sind die Vorsprünge (2131) so konfiguriert, um irgendwelche Anhaftungen an der Wand des Schaftlochs (1222) abstreifen.

2. Winkeleinstellmechanismus (M) nach Anspruch 1, wobei das Haltegestell (1231) eine Kreismitte hat, die sich auf der Achse (C) befindet, das Haltegestell (1231) einen Mittelwinkel (C1231) hinsichtlich der Achse (C) hat, und der Mittelwinkel (C1231) im Wesentlichen gleich zum Winkelbereich ist, und wobei der biegsamen Bügel (2) aus einem einzigen Material gefertigt ist, und der Winkelreichweite innerhalb einer Reichweite von 15 Grad bis zu 30 Grad ist.

3. Winkeleinstellmechanismus (M) nach Anspruch 1, wobei der Montageschlitz (120) eine Schlitzöffnung (1221) an einem Ende der begrenzenden Seitenplatte (122) aufweist, die vom begrenzenden Endabschnitt (123) weg ist, wobei die begrenzende Platte (122) eine Führungsnut (1223) hat, die von ihrem Ende zum Schaftloch (1222) hin vertieft ist, die Führungsnut (1223) mit der Schlitzöffnung (1221) in räumlicher Verbindung ist und die Rotationswelle (213) durch Bewegen entlang der Führungsnut (1223) in das Schaftloch (1222) eingebaut wird, und wobei ein Abstand zwischen der Führungsnut (1223) und dem passenden Abschnitt (1211) in einer Richtung von der Schlitzöffnung (1221) zum Schaftloch (1222) sich allmählich verringert.

4. Winkeleinstellmechanismus (M) nach Anspruch 3, wobei das Montagesegment (21) einen L-förmigen Arm (212) aufweist, der in den Montageschlitz (120) eingeführt ist, der Eingriffszahn (2121) an einem freien Ende des L-förmigen Arms (212) angeordnet ist und die Rotationswelle (213) an einer Innenseite des L-förmigen Arms (212) gebildet ist.

5. Winkeleinstellmechanismus (M) nach Anspruch 4, wobei das Montagesegment (21) ein Abschirmblech (211) aufweist, der L-förmige Arm (212) mit der Abschirmblech (211) so verbunden ist, dass sie die begrenzende Seitenplatte (122) gemeinsam beidseitig abdecken, und das Abschirmblech (211) deckt das Schaftloch (1222) ab.

6. Winkeleinstellmechanismus (M) nach Anspruch 5, wobei der L-förmige Arm (212) ein Durchgangsloch (2122) hat, das neben dem Eingriffszahn (2121) angeordnet ist, und einen Abstand zwischen dem Durchgangsloch (2122) und der Rotationswelle (213) mindestens das Dreifache eines Abstands zwischen dem Durchgangsloch (2122) und dem Eingriffszahn (2121) ist.

7. Schutzbrille, die aufweist:
eine Linse (11);
zwei Winkeleinstellmechanismen (M) nach irgendeinem der Ansprüche 1 bis 6, wobei zwei Seitenrahmen (12) jeweils an zwei gegenüberliegenden Seiten der Linse (11) verbunden sind, wobei zwei Seiten der zwei Seitenrahmen (12), die voneinander weg sind, jeweils den Montageschlitz (120) des entsprechenden Winkeleinstellmechanismus (M) haben, und das Haltegestell (1231) neben der Linse (11) angeordnet ist; und
einen oberen Rahmen (13), der mit der Linse (11) und den zwei Seitenrahmen (12) verbunden ist,
wobei die Linse (11), die zwei Seitenrahmen (12) und der obere Rahmen (13) gemeinsam einen Augenschutzraum (S) definieren,
wobei jeder der zwei Seitenrahmen (12) eine Hauptplatte (121) aufweisen, die begrenzende Seitenplatte (122) des zugehörigen Winkeleinstellmechanismen (M) der Hauptplatte (121) zugewandt ist, und wobei der begrenzende Endabschnitt (123) der zugehörigen Winkeleinstellmechanismen (M) die Hauptplatte (121) und die begrenzende Seitenplatte (122) verbindet.

8. Schutzbrille nach Anspruch 7, wobei jede der zwei Seitenrahmen (12) eine begrenzende obere Platte (124) aufweist, die komplanar mit dem oberen Rahmen (13) ist, und wobei in jedem der zwei Seitenrahmen (12), die begrenzende, obere Platte (124) ganzheitlich mit der Hauptplatte (121), dem begrenzenden Endabschnitt (123), und der begrenzenden Seitenplatte (122) verbunden ist.

9. Schutzbrille nach Anspruch 7, wobei jeder der beiden biegsamen Bügel (2) aus einem einzigen Material hergestellt ist, und wobei sich das flexible Segment (22) jedes der beiden biegsamen Bügel (2) gebogen vom Montagesegment (21) zur Linse (11) hin sich erstreckt, so dass die flexiblen Segmente (22) der zwei biegsamen Bügel (2) sich gegenseitig berühren, und wobei die Linse, die zwei Seitenrahmen (12), und der obere Rahmen (13) lichtdurchlässig und als eine einzelne, ein-stückige Struktur geformt sind, und die Winkelreichweite innerhalb einer Reichweite von 15 Grad bis zu 30 Grad ist.

## Revendications

1. Mécanisme (M) de réglage d'angle de lunettes de sécurité, le mécanisme comprenant :
une fente d'assemblage (120) d'un cadre latéral (12) des lunettes de sécurité, une crémaillère de retenue (1231) étant agencée dans la fente d'assemblage (120), la crémaillère de retenue (1231) prenant une forme en arc ; et
une branche souple (2) formée entièrement comme une structure en une seule pièce et comprenant un segment d'assemblage (21) relié pivotant à la fente d'assemblage (120) le long d'un axe (C), lequel est horizontal dans une position de port normal des lunettes de sécurité, et comportant une dent d'entrée en prise (2121), et un segment flexible (22) s'étendant à partir du segment d'assemblage (21) ;
dans lequel la dent d'entrée en prise (2121) du segment d'assemblage (21) est introduite dans la fente d'assemblage (120) et entre en prise avec la crémaillère de retenue (1231), et le segment d'assemblage (21) est rotatif par rapport à la fente d'assemblage (120) selon une plage d'angles, de sorte que la dent d'entrée en prise (2121) entre en prise avec la crémaillère de retenue (1231) et soit mobile d'une extrémité de la crémaillère de retenue (1231) à une autre extrémité de la crémaillère de retenue (1231) ;
dans lequel la fente d'assemblage (120) comprend une planchette latérale de limitation (122), une partie d'accouplement (1211) faisant face à la planchette latérale de limitation (122), une partie d'extrémité de limitation (123) servant à relier la partie d'accouplement (1211) et la planchette latérale de limitation (122), et dans lequel la crémaillère de retenue (1231) est formée sur la partie d'extrémité de limitation (123), la planchette latérale de limitation (122) comporte un trou (1222) d'arbre, et le segment d'assemblage (21) comporte un arbre tournant (213) assemblé au trou (1222) d'arbre ; et
dans lequel l'arbre tournant (213) comporte une pluralité de parties saillantes (2131) venant en butée contre une paroi du trou (1222) d'arbre, et lorsque le segment d'assemblage (21) est mis en rotation par rapport à la fente d'assemblage (120), les parties saillantes (2131) sont conçues pour éliminer par grattage des adhésions quelconques sur la paroi du trou (1222) d'arbre.

2. Le mécanisme (M) de réglage d'angle selon la revendication 1, dans lequel la crémaillère de retenue (1231) comporte un centre de cercle situé au niveau de l'axe (C), la crémaillère de retenue (1231) comprend un angle central (C1231) par rapport à l'axe (C), et l'angle central (C1231) est sensiblement égal à la plage d'angles, et dans lequel la branche souple (2) est constituée d'un seul matériau, et la plage d'angles est comprise entre 15 degrés et 30 degrés.

3. Le mécanisme (M) de réglage d'angle selon la revendication 1, dans lequel la fente d'assemblage (120) comporte une ouverture (1221) de fente agencée sur une extrémité de la planchette latérale de limitation (122) éloignée de la partie d'extrémité de limitation (123), dans lequel la planchette latérale de limitation (122) comporte une rainure de guidage (1223) évidée à partir de son extrémité vers le trou (1222) d'arbre, la rainure de guidage (1223) est en communication spatiale avec l'ouverture (1221) de fente, et l'arbre tournant (213) est assemblé dans le trou (1222) d'arbre au moyen de son déplacement le long de la rainure de guidage (1223), et dans lequel une distance entre la rainure de guidage (1223) et la partie d'accouplement (1211) diminue progressivement dans une direction allant de l'ouverture (1221) de fente au trou (1222) d'arbre.

4. Le mécanisme (M) de réglage d'angle selon la revendication 3, dans lequel le segment d'assemblage (21) comprend un bras en L (212) introduit dans la fente d'assemblage (120), la dent d'entrée en prise (2121) est agencée sur une extrémité libre du bras en L (212), et l'arbre tournant (213) est formé sur un côté interne du bras en L (212).

5. Le mécanisme (M) de réglage d'angle selon la revendication 4, dans lequel le segment d'assemblage (21) comprend une feuille de blindage (211), le bras en L (212) est relié à la feuille de blindage (211) de manière à encadrer conjointement la planchette latérale de limitation (122), et la feuille de blindage (211) recouvre le trou (1222) d'arbre.

6. Le mécanisme (M) de réglage d'angle selon la revendication 5, dans lequel le bras en L (212) comporte un trou traversant (2122) agencé adjacent à la dent d'entrée en prise (2121), et une distance entre le trou traversant (2122) et l'arbre tournant (213) est au moins trois fois une distance entre le trou traversant (2122) et la dent d'entrée en prise (2121).

7. Lunettes de sécurité, comprenant :
un verre de lunettes (11) ;
deux mécanismes (M) de réglage d'angle selon l'une quelconque des revendications 1 à 6, dans lesquels deux cadres latéraux (12) sont reliés respectivement à deux côtés opposés du verre de lunettes (11), et dans lesquels deux côtés des deux cadres latéraux (12) éloignés l'un de l'autre comportent chacun la fente d'assemblage (120) des mécanismes (M) de réglage d'angle correspondants, et la crémaillère de retenue (1231) est agencée adjacente au verre de lunettes (11) ; et
un cadre supérieur (13) relié au verre de lunettes (11) et aux deux cadres latéraux (12), le verre de lunettes (11), les deux cadres latéraux (12) et le cadre supérieur (13) définissant conjointement un espace (S) de protection des yeux,
dans lesquelles chacun des deux cadres latéraux (12) comprend une planchette principale (121), la planchette latérale de limitation (122) des mécanismes (M) de réglage d'angle correspondants faisant face à la planchette principale (121), et dans lesquelles la partie d'extrémité de limitation (123) des mécanismes (M) de réglage d'angle correspondants relie la planchette principale (121) et la planchette latérale de limitation (122).

8. Les lunettes de protection selon la revendication 7, dans lesquelles chacun des deux cadres latéraux (12) comprend une planchette supérieure de limitation (124) coplanaire avec le cadre supérieur (13), et dans lesquelles, dans chacun des deux cadres latéraux (12), la planchette supérieure de limitation (124) est reliée en une seule pièce à la planchette principale (121), à la partie d'extrémité de limitation (123) et à la planchette latérale de limitation (122).

9. Les lunettes de protection selon la revendication 7, dans lesquelles chacune des deux branches souples (2) est constituée d'un seul matériau, et dans lesquelles le segment flexible (22) de chacune des deux branches souples (2) s'étend de manière incurvée du segment d'assemblage (21) vers le verre de lunettes (11), de sorte que les segments flexibles (22) des deux branches souples (2) se mettent en contact l'un avec l'autre, et dans lesquelles le verre de lunettes, les deux cadres latéraux (12) et le cadre supérieur (13) sont perméables à la lumière et sont formés entièrement comme une structure en une seule pièce, et la plage d'angles est comprise entre 15 degrés et 30 degrés.
